# EUROPÄISCHE PATENTSCHRIFT

(11) **EP 1 385 002 B1**
(45) Veröffentlichungstag und Bekanntmachung des Hinweises auf die Patenterteilung: **12.09.2007**
(21) Anmeldenummer: 03090150.8
(22) Anmeldetag: 21.05.2003
(51) Int. Cl.: G01N 33/543, C12Q 1/00

(54) **Biosensor mit kompartimentiertem Reaktions- und Messkammeraufsatz**
Biosensor with compartmentalised reaction and measurement chamber attachment
Biocapteur avec un accessoire compartimenté comprenant une chambre de réaction et de mesure

(30) Priorität: 25.07.2002 DE 10234564
(43) Veröffentlichungstag der Anmeldung: 28.01.2004
(73) Patentinhaber: SensLab - Gesellschaft zur Entwicklung und Herstellung bioelektrochemischer Sensoren mbH, 04347 Leipzig (DE)
(72) Erfinder: Gründig, Bernd Dr., 04318 Leipzig (DE); Hänsler, Marion, Dr. habil., 04229 Leipzig (DE); Kopinke, Holm Dipl.-Ing., 04357 Leipzig (DE); Wedig, Heiko Dipl.-Ing., 04109 Leipzig (DE); Hellmich, R., Dipl.-Chem., 04157 Leipzig (DE)
(74) Vertreter: Ziebig, Marlene

(56) Entgegenhaltungen:
- EP-A- 1 113 264
- EP-A- 1 203 956
- WO-A-01/27627
- DE-A- 10 001 116
- US-A- 5 885 527
- US-B1- 6 368 478

## Beschreibung

Die Erfindung betrifft einen Biosensor, der aus einem planaren Sensor oder Teststreifen 1 und einem kompartimentierten Reaktions- und Messkammeraufsatz 2 besteht, die form- und kraftschlüssig miteinander verbunden sind. Der Biosensor besitzt streng reproduzierbare, kostengünstige und massenproduktionsfähige Eigenschaften zur Ausmessung von Substanzen oder Enzymaktivitäten in geringsten Probevolumina.

Für den erfindungsgemäßen Biosensor mit Reaktions- und Messkammeraufsatz 2 wird ein zweifach oder mehrfach durch Kanäle kompartimentierter Reaktions- und Messkammeraufsatz 2, vorzugsweise aus Kunststoff, mit einer Innengeometrietiefe im Mikrometerbereich genutzt, der photolithographisch, im Mikrospritzguss-, im Tiefzieh- oder Heißprägeverfahren hergestellt wird. In seinen Ausführungsformen wird der Formkörper aus Kunststoff mit der planaren Oberfläche des Sensors 1 irreversibel verbunden. Aufgrund seiner inneren Geometrie und Oberflächenbeschichtung erfolgt eine definierte Probeaufnahme, ein kontrolliertes Beschleunigen, Verzögern oder Abstoppen des Probeflusses.

Der erfindungsgemäße Biosensor ist besonders geeignet für die kostengünstige Realisierung von Enzym- und Affinitätssensoren, insbesondere voltammetrischer Enzym- und Affinitätssensoren für den einmaligen Gebrauch.

Mit der Nutzung von Sensoren für den Einmalgebrauch und Teststreifen auf der Grundlage enzymatischer und affinitätsbasierender Detektionsprinzipien für schnelle und kostengünstige Vor-Ort-Tests, aber auch quantitativer Messungen sind Sensorstrukturen erforderlich, die einerseits eine möglichst richtige und reproduzierbare Messung sichern, andererseits aber kostengünstig herstellbar sein müssen. Darüber hinaus sollte die erforderliche Probemenge so gering als möglich sein. Diese Kriterien erfordern ein streng reproduzierbares Liquid-Handling der Probe.

Quantitative Vor-Ort-Messungen, die einfach durchzuführen sind und neben dem Sensorelement oder Teststreifen nur ein Handmessgerät zur Signalauswertung als "Signalausleseeinheit" benötigen, sind als so genannte "Einmal"- oder "Wegwerf"-Teststreifen oder -Sensoren bekannt. Für Messsysteme, die eine enzymatisch-voltammetrische Indikation nutzen und die insbesondere für den Einmalgebrauch in der medizinischen Diagnostik, im Point of Care- oder Home Care-Bereich verwendet werden, sind eine Vielzahl technischer Lösungen bekannt.

Die bekannten technischen Lösungen bestehen prinzipiell aus einem Kunststoffsupport, auf den eine Zwei- oder Dreielektrodenanordnung aus Kohlenstoffpasten oder einer metallisch dünnen Schicht oder aus Metallfolien mittels Siebdruck, Sputtern oder durch Aufkleben respektive aufgebracht wurde. Unmittelbar über der Elektrodenanordnung befindet sich eine Reaktionsschicht, die einen Elektronenmediator, eine Oxidoreduktase sowie Additive zur Stabilisierung und schnellen Benetzbarkeit enthält. Als Oxidoreduktasen werden Oxidasen, PQQ-abhängige Dehydrogenasen oder NAD⁺-abhängige Dehydrogenasen und als Mediatoren beispielsweise Chinone, chinoide Redoxfarbstoffe oder redoxaktive Metallkomplexe verwendet. Eine Reihe technischer Lösungen sehen zusätzlich Schichten mit Filterwirkung gegenüber Blutzellen und anderen im Blut enthaltenen und die Messung beeinflussenden Substanzen vor (z.B. US 5,628,890, EP 0874984, WO 99/30152). Die Nachweisreaktion erfolgt durch eine voltammetrisch kontrollierte Oxidation des aus der enzymatischen Reaktion mit dem Blutanalyten zuvor reduzierten Redoxmediators. Eine bekannte technische Lösung benutzt das Prinzip der spektroelektrochemischen Dünnschichtzelle mit coulometrischem Nachweis des enzymatischen Gesamtstoffumsatzes an reduziertem Mediator bzw. Analyten in der Messzelle (US 6,338,790; Feldman et al., Diabetes Technology Therapeutics 2 (2000) 2, 221-229).

Die Probe wird entweder direkt auf die Schichtenfolge gegeben oder mittels eines Kapillareffekts in die Reaktionsschicht über der Elektrodenanordnung gesaugt (EP 274215, US 490,042). Dazu wird ein Kapillarspalt zur Probeaufnahme unmittelbar über der Elektrodenanordnung geschaffen. Technische Lösungen zum Aufbau einer kapillaren Messkammer bzw. eines Kapillarspaltes beschreiben beispielsweise die Verwendung von Spacern seitlich der Elektrodenanordnung. Als Spacer dienen verklebte Kunststoffzwischenlagen, doppelseitig mit einem Kleber versehene Folien oder starke Klebeschichten, die im Sieb- oder Schablonendruck aufgetragen werden. Auf die Spacerschicht wird im Anschluss eine Deckschicht aufgebracht, so dass eine Ein- und Austrittsöffnung für die Probeflüssigkeit generiert wird (z.B. US 5,120,420, US 5,645,709). Eine weitere technische Lösung sieht die Ausbildung einer konkaven Wölbung in der Abdeckung vor, die nach dem Verkleben mit dem Support einen Kapillarspalt erzeugt (US 5,759,364).

In den beschriebenen technischen Lösungen wird die Probe aufgrund der Kapillarwirkung auf die sensitive Fläche des Sensors transportiert. Eine große Austrittsöffnung führt zur Aufhebung der Kapillarwirkung, so dass die Probeaufnahme damit beendet wird. Varianten nutzen als Austrittsöffnung beispielsweise eine Öffnung in der Abdeckung oder eine Öffnung im Support des Sensors.

Auf diese Weise wird auch das Gesamtvolumen der Probe festgelegt.
Die Kapillarwirkung wird durch die Verwendung hydrophilisierter Oberflächen (US 5,759,364) und das zusätzliche Einbringen hydrophiler Polymere und sorptiver Polymere bzw. hydrophilisierter Gewebe in ihrer Wirkung in verschiedenen technischen Lösungen verbessert (US 5,997,817, US 5,708,247).

Gemeinsam ist den beschriebenen Systemen, dass bei dem Prinzip des kapillaren Probetransports die auszumessende Probe über einen Kapillarspalt aufgesaugt wird und die kapillarkraftbedingte Probeaufnahme mehr oder weniger reproduzierbar an einer Austrittsöffnung abreißt, wobei Reste der Probe am Rande der Austrittsöffnung austreten können. Darüber hinaus beschränken sich die Möglichkeiten einer gegebenenfalls erforderlichen Probevorbehandlung auf den Bereich der Messkammer selbst, in der die eigentliche Indikationsreaktion jedoch mit der Probeaufnahme sofort beginnt.

Bekannte technische Lösungen zum Aufbau immunochemischer Teststreifen bzw. Sensoren oder Tests, die Affinitätsreaktionen nutzen, basieren in aller Regel auf nacheinander überlappend angeordneten, filtrierenden und gut saugfähigen natürlichen oder synthetischen Polymermembrangerüstschichten aus zelluloseartigen Materialien wie Zellulosenitrat, Zelluloseester und regenerierter Zellulose oder Materialien aus modifizierten Polyamiden oder modifiziertem Polyethersulfon. Dadurch werden vertikal (z.B. DE 19622458) oder horizontal verlaufende Fließstrecken realisiert (z.B. DE 4037724, US 6,221,238). Neben einer Separation der Probe kann sowohl die immunochemische Reaktion als auch die sekundäre Nachweisreaktion in qualitativer Form durch Visualisierung einer Farbreaktion oder Aufkonzentrierung metallischer Mikropartikel oder in quantitativer Form mittels elektrochemischer Detektion oder optischer Detektion erfolgen. Von Nachteil sind das vergleichsweise große erforderliche Probevolumen und das ungünstige Verhältnis von verfügbarem Probevolumen zu gegebener Festphasenoberfläche.

Bekannt ist auch ein Mikrofluidiksystem, das einen verzweigten Glaskanalchip in Kombination mit einem amperometrischen Dickschichtsensor verwendet. Dabei wird durch Anlegen einer gepulsten Spannung der Transport der getrennt deponierten Immunoreagenzien sowie von Probe-, Puffer- und Substratflüssigkeit initiiert. Die vorteilhafte Mikrofluidik ermöglicht eine Nachweisgrenze im femtomolaren Bereich, allerdings fehlt die adäquate Integration von Fluidik und Sensorelement sowie ein einfaches Liquidhandling.

In EP-A-1 203 956 ist ein Biosensor beschrieben, welcher aus Ober- und Unterteil besteht, die gemeinsam einen Kapillarspalt bilden, der die flüssige Probe zur Reaktionsschicht transportiert und der verschiedene Höhen aufweist.

Aufgabe der Erfindung war es deshalb, einen Biosensor bereitzustellen, der die oben genannten Nachteile sowohl bei den bekannten Einmal-Enzym-Sensoren als auch Einmal-Affinitätssensoren vermeidet und technologisch kostengünstig, einfach und streng reproduzierbar herstellbar ist. Darüber hinaus soll der Biosensor eine einfache Handhabung ermöglichen, ein minimales und ein streng reproduzierbares und definiertes Probevolumen aufnehmen und die Bestimmung sowohl von Substanzen als auch von Enzymaktivitäten ermöglichen.

Die Aufgabe der Erfindung wird gemäß den unabhängigen Ansprüchen gelöst. Die Unteransprüche stellen bevorzugte Ausführungsvarianten dar.

Es wurde gefunden, dass ein Biosensor mit Reaktions- und Messkammeraufsatz 2 mit streng reproduzierbaren, kostengünstigen und massenproduktionsfähigen Eigenschaften zur Ausmessung von Substanzen oder Enzymaktivitäten in geringsten Probevolumina erhalten wird, wenn der Reaktions- und Messkammeraufsatz 2, der mittels photolithographischen Verfahrens, Laserstrukturierung, Mikrospritzguss, Tiefziehverfahren oder Heißprägeverfahren aus Kunststoff hergestellt werden kann, zweifach oder mehrfach durch Kanäle kompartimentierte Kammern oder Räume aufweist. Die Kanäle stellen Verbindungen zu den einzelnen, nacheinander angeordneten Kammern bzw. Räumen dar. Die Innengeometrietiefe der Kanäle und Kammern liegt im Mikrometerbereich. Der Formkörper der Reaktions- und Messkammer 4 ist in seinen verschiedenen Ausführungsformen immer mit der planaren Oberfläche 1 des Biosensors durch eine form- oder kraftschlüssige Verbindung irreversibel verbunden.
Die Tiefe der photolithographisch strukturierten, im Mikrospritzgussverfahren hergestellten, tiefgezogenen oder heißgeprägten Geometrie des Innenraumes des Reaktions- und Messkammeraufsatzes 2 kann sich sowohl innerhalb des Kompartiments als auch von Kompartiment zu Kompartiment ändern und beträgt zwischen 10 µm und 300 µm. Als Material sind Folien oder Platten aus Polycarbonat, Polymethylmethacrylat, Polystyrol oder Polyvinylchlorid mit Dicken zwischen 0,1 mm und 2,0 mm geeignet.

Aufgrund der inneren Geometrie des Reaktions- und Messkammeraufsatzes 2, die sowohl sprunghafte Querschnittsänderungen von Kanal zu Kompartiment und von Kompartiment zu Kanal als auch kontinuierliche Änderungen innerhalb des Kompartiments aufweist und darüber hinaus mit Oberflächenmodifikationen innerhalb bestimmter Geometrieabschnitte versehen wird, gelingt eine definierte Probeaufnahme, deren verzögerter Durchfluss oder Verbleib in einer der Kammern, so dass ein kontrollierter Probefluss vom Probeaufnahmekanal 3 über gegebenenfalls verschiedene Probeaufnahme-, Vorbereitungs-, Affinitätsreaktions-, Substratdepot-, Reaktions- und Messkammern bis zu einem Probeauffangraum 6 realisiert werden kann.

Im einfachsten Fall besteht der Reaktions- und Messkammeraufsatz 2, der mittels form- oder kraftschlüssiger Verbindung auf der ebenen Oberfläche des Sensors 1 fixiert ist, aus einem Probeaufnahmekanal 3 mit einem Volumen zwischen 0,005 µl und 0,05 µl, einer gemeinsamen Reaktions- und Messkammer 4 mit einem Volumen zwischen 0,1 µl und 1,0 µl, einem Probestoppkanal 5 mit einem Volumen zwischen 0,005 µl und 0,05 µl, der den kapillaren Probefluss blockiert und einem Probeauffangraum 6 mit einem Volumen von mindestens 10 µl. Der Probeaufnahmekanal 3, dessen Wandungen oder Basisfläche mittels eines Tensides hydrophilisiert werden oder der ein hydrophilisiertes poröses Füllmaterial enthält, und dessen Querschnitt eine Tiefe zwischen 10 µm und 50 µm aufweist, verfügt über eine Eintrittsöffnung. Die sich dem Probeaufnahmekanal 3 anschließende Reaktions- und Messkammer 4, die direkt über dem Messfenster bzw. der Elektrodenanordnung 1b 1d des planaren Sensors 1 positioniert ist, kann sich in der Tiefe und / oder Breite ihres Querschnittes gegenüber der Tiefe und / oder Breite des Querschnitts des Eintrittskanals 3 entweder sprunghaft beispielsweise auf das Doppelte bis Zwanzigfache vergrößern oder kann von der Eintritts- bis zur Austrittsöffnung der Reaktions- und Messkammer 4 kontinuierlich auf das Doppelte bis Zwanzigfache ansteigen. Danach folgt ein so genannter Probestoppkanal 5, der in der Tiefe und / oder Breite seines Querschnitts beispielsweise um die Hälfte bis Zwanzigfache geringer ist als die Tiefe und / oder Breite des Austrittsöffnungsquerschnitts der Reaktions- und Messkammer 4 und dessen innere Wandungen mittels längerkettigen Kohlenwasserstoffen oder Derivaten davon oder mittels eines auf organischen Lösungsmitteln basierenden Haftklebers modifiziert sind oder der ein hydrophobisiertes sorbtives Füllmaterial enthält, und eine kapillarkrafthemmende Wirkung auf die Probeflüssigkeit ausübt. Dieser Probestoppkanal 5 mündet in einem so genannten Probeauffangraum 6, der eine Tiefe zwischen 100 µm und 300 µm aufweist.
Als poröses Füllmaterial werden porös strukturierte natürliche oder künstliche Materialien aus Zellulose, Zellulosederivaten, Polyurethanen, Zeolithen, Silikaten oder Gemischen davon verwendet.
Die Probe wird aufgrund der kapillarkraftfördernden Geometrie des Probeaufnahmekanals 3 bzw. dessen sorptiven Füllmaterials sehr schnell aufgenommen und bis in die Reaktions- und Messkammer 4 weitergeleitet. Durch die Querschnittserweiterung der Reaktions- und Messkammer 4 und die kapillarkrafthemmende Wirkung des nachfolgenden Probestoppkanals 5 kommt der Probefluss zum Stillstand und verbleibt damit in der Reaktions- und Messkammer 4. Sollte lange nach Beendigung der Messung die Probe dennoch den Probestoppkanal 5 durchbrechen, gelangt die Probe in den Probeauffangraum 6, der in seinen inneren Abmaßen eine abrupte Vergrößerung der Tiefe / und oder Breite des Querschnitts gegenüber der Tiefe / und oder Breite des Querschnitts des Probestoppkanals 5 aufweist und vom Volumen so groß ist, dass die Kapillarkraftwirkung aufgehoben wird und der Probefluss in dieser Kammer 6 zum Stillstand kommt. Aufgrund der Größe dieses Raumes 6, der mit seinem Volumen von mindestens 10 µl über die gesamte Sensorfläche 1 reicht und am Ende eine Öffnung zum Luftdruckausgleich aufweist, verbleibt die Probe mit einem Volumen kleiner 1 µl in diesem Raum 6.
Gegebenenfalls kann zwischen dem Probeaufnahmekanal 3 und der Reaktions- und Messkammer 4 eine Probekonditionierungskammer oder Probevorbehandlungskammer 7 angeordnet sein, die in ihren Eigenschaften denen der Reaktions- und Messkammer 4 analog ist und aufgrund einer entsprechenden Vorbefüllung beispielsweise zur Hämolyse, zur Anhebung oder Absenkung des pH-Wertes der Probe, zur Probepufferung, zur Separation von Probebestandteilen, zur Eliminierung von Interferenzen oder zur Aufnahme von Co-Reaktanden in die Probe, die für die nachfolgende Reaktion, wie sie beispielsweise zur Bestimmung von Enzymaktivitäten oder Cofaktor-abhängiger Oxidoreduktasereaktionen erforderlich sind, dient.
Die beschriebene Ausführungsform ist besonders für voltammetrische Enzymsensoren zum Einmalgebrauch geeignet.

Eine weitere Ausführungsform des erfindungsgemäßen Biosensors ist dadurch gekennzeichnet, dass es sich um einen mehrfach durch Kanäle kompartimentierten Reaktions- und Messkammeraufsatz 2 handelt. Diese in Kammer- und Kanalgeometrien, Material, Oberflächenmodifizierung und Fixierung mit der vorigen Ausführungsform identische Variante besteht jedoch neben einem Probeaufnahmekanal 3 mit einem Volumen zwischen 0,02 µl und 0,05 µl, aus einer Probeaufnahme- und Affinitätsreaktionskammer 8 mit einem Volumen zwischen 2 µl und 20 µl, einer Substrat- und Pufferdepotkammer 10 mit einem Volumen zwischen 0,1 *µ*l und 5 *µ*l, einer Fängerzonen-und Messkammer 4 mit einem Volumen zwischen 0,1 *µ*l und 5 *µ*l und einem Probeauffangraum 6 mit einem Volumen von mindestens 10 µl. Diese Kompartimente sind durch kanalartige Übergänge mit Volumen zwischen 0,02 µl und 0,05 µl, die den Durchtritt der Probe beschleunigen, verzögern oder stoppen, miteinander verbunden. Die Realisierung der kapillarkraftfördernden oder -hemmenden Wirkung in diesen Übergangskanälen erfolgt, wie in der vorangegangenen Ausführungsform beschrieben. Bei kurzzeitigem Kontakt mit Probeflüssigkeit erfolgt über die Eintrittsöffnung des Probeaufnahmekanals 3 die rasche Aufnahme der Probe, die zur Befüllung der Probeaufnahmekammer 8, die gleichzeitig die Affinitätsreaktionskammer 8 darstellt, führt. Mit der Befüllung der Probeaufnahme- und Affinitätsreaktionskammer 8 wird zunächst ein definiertes Probevolumen realisiert. Darüber hinaus enthält diese Kammer sowohl an ihren Wandungen immobilisiert Antikörper, Antigene/Haptene, DNA-Abschnitte oder Oligonukleotide als auch den Analyten in Form eines Antigens/Haptens, Antikörpers, DNA-Abschnitts oder Oligonukleotids, der mit einem Enzym oder einem optisch oder elektrochemisch aktiven Molekül konjugiert ist und bei Anwesenheit der Probe in der Lösung frei diffusionsfähig ist. Da die inneren Wandungen des Verbindungskanals 9 zur Substrat- und Pufferdepotkammer 10 in einem bestimmten Ausmaß hydrophobisiert sind oder ein hydrophobisiertes poröses Füllmaterial enthalten und damit eine kapillarkrafthemmende Wirkung auf die Probeflüssigkeit ausüben, verbleibt die Probeflüssigkeit zunächst in der Probeaufnahmekammer 8. Der in der Probe enthaltene Analyt kann in Konkurrenz mit der konjugierten Form des in der Probeaufnahme- und Affinitätsreaktionskammer 8 vorliegenden Analyten einer Affinitätsreaktion mit dem immobilisierten Erkennungsmoleküls in Form eines Antikörpers, Antigens/Haptens, DNA-Abschnitts oder Oligonukleotids eingehen. Nach definierter Inkubationszeit bzw. definierter Verzögerung des Probeflusses durch den Verbindungskanal 9 zur Substrat- und Pufferdepotkammer 10 gelangen die mit dem Marker konjugierten Analytmoleküle, die keine Bindung mit den immobilisierten Erkennungsmolekülen eingehen konnten, durch den Probefluss in die Substrat- und Pufferdepotkammer 10 und von dort in die Messkammer 4, die direkt über der sensitiven Fläche 1b, 1d des Sensors oder Teststreifens fixiert ist. In der Substrat- und Pufferdepotkammer 10 wird die Probe gegebenenfalls auf das pH Optimum der Nachweisreaktion des Markermoleküls eingestellt und mit dem entsprechenden Substrat und Cosubstraten angereichert, so dass die sekundäre Nachweisreaktion damit startet. An den Wandungen der Messkammer 4 sind Fängermoleküle immobilisiert, die das Konjugat binden und damit in der Messzelle anreichern. Das Markermolekül tritt direkt oder über ein Reaktionsprodukt mit der sensitiven Fläche 1b, 1d des Sensors oder Teststreifens in Wechselwirkung und der Sensor oder Teststreifen 1 generiert ein analytabhängiges Messsignal. Der nachfolgende Probestoppkanal 5 ist hydrophobisiert bzw. enthält ein stark hydrophobisiertes sorbtives Füllmaterial und bewirkt, dass die Probe innerhalb der Messkammer 4 verbleibt. Sollte nach Beendigung der Messung die Probe den Probestoppkanal 5 durchbrechen, gelangt die Probe in die Probeauffangkammer 6, in der der Probefluss zum Stillstand kommt.
Aufgrund des geringen erforderlichen Probevolumens, des vorteilhaften Verhältnisses von Probevolumen zu Festphasenoberfläche und des einfachen und reproduzierbaren Flüssigkeitshandlings ist diese Ausführungsform besonders für die Realisierung hochsensitiver Affinitätssensoren zum Einmalgebrauch geeignet.

Damit wird ein Biosensor bereitgestellt, der für die Ausführungsvariante als Enzymsensor die Probe, deren Volumen kleiner als ein Mikroliter ist, durch definierte und unterschiedliche Kapillarkraftwirkung der Kanäle bzw. der Kompartimentgeometrie und -oberfläche oder durch den Flüssigkeitstransport über sich in ihren Sorptionseigenschaften unterscheidende poröse Füllmaterialien in den Kanälen und Kompartimenten kontrolliert und reproduzierbar aufnimmt, transportiert und im Messkammerbereich belässt, wobei gegebenenfalls die Reaktionskammer als eine Probevorbereitungs- bzw. Konditionierungskammer separat ausgebildet und der Messkammer vorgeschaltet ist.

In der Ausführungsvariante als Affinitätssensor wird die Probe, deren Volumen bis zu 20 µl beträgt, durch definierte und unterschiedliche Kapillarkraftwirkung der Kanäle bzw. der Kompartimentgeometrie und -oberfläche oder durch den Flüssigkeitstransport über sich in ihren Sorptionseigenschaften unterscheidende sorptive Füllmaterialien in den Kanälen und Kompartimenten kontrolliert und reproduzierbar aufgenommen, durchläuft eine Reihe von Kompartimenten bzw. Kammern und endet schließlich im Messkammerbereich.

### Ausführungsbeispiele

Der erfindungsgemäße Biosensor wird durch nachfolgende Ausführungsbeispiele und Zeichnungen näher erläutert.

### Beispiel 1:

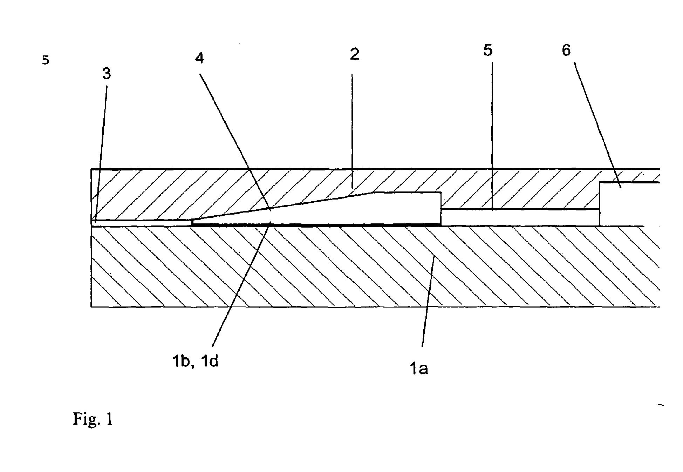

Erfindungsgemäßer Biosensor für den Einmalgebrauch zum Nachweis von Lactat. Zur Erläuterung dienen Fig. 1 bis Fig. 3.

Auf einen PVC Kunststoffsupport 1 mit einer Dicke von 0,6 mm werden in Zehnernutzen mittels Siebdruck nacheinander Elektrodenflächen 1b bzw. Kohlenstoffkontaktbahnen 1c (Acheson PE 401, Acheson NL), Isolationslack 1e (240 SB, ESL Europe) und Kleber (D 159, Kiwoprint) wie in Fig. 1 dargestellt aufgedruckt und jeweils bei 70°C gehärtet. Die Geometrie des Kleberfilms ist identisch mit der Geometrie der Isolationslackschicht 1e. Die Abmessungen eines Sensors sind 6 mm x 36 mm (B x L). Die Einzelflächen von Arbeits-, Referenz- und Gegenelektrode, die nacheinander angeordnet sind und die eigentliche Elektrodenfläche 1b ausmachen, betragen jeweils 1 mm². Mittels eines Dispensers werden 0,3 µl einer Reaktionslösung bestehend aus 1,3 Units Lactatoxidase (Fluka), 45 µg Ferricyanid (Sigma), 1,6 µg Triton X 100 (Sigma) und 1,5 µg mikrokristalliner Zellulose (Aldrich) als Reaktionsschicht 1d in 0,02 µl Dispensierschritten gleichmäßig über die gesamte Elektrodenfläche verteilt aufgetragen.
Für den Reaktions- und Messkammeraufsatz 2 wird eine PVC-Folie von 0,2 mm Dicke mittels einer Stahlpatrize heißgeprägt. Die als Zehnernutzen hergestellte Patrize weist Erhebungen für einen Probeaufnahmekanal 3, eine Reaktions-und Messkammer 4, einen Probestoppkanal 5, und einen Probeauffangraum 6 auf. Dementsprechend wurden Kanäle und Kompartimente mit folgenden Geometrien realisiert: Probeaufnahmekanal 3: 50 µm x 100 µm x 1 mm (H x B x L), Reaktions- und Messkammer 4: 100 µm x 1 mm x 3 mm (H x B x L), Probestoppkanal 5: 100 µm x 100 µm x 1 mm (H x B x L) und Probeauffangraum 6: 250 µm x 3 mm x 25 mm (H x B x L).
Mittels Dispenser werden nacheinander 20 nl einer 0,1%igen wässrigen Lösung aus Triton X 100 in den Probeaufnahmekanal 3 und 20 nl einer 2%igen Lösung aus Paraffinwachs (Aldrich) in den Probestoppkanal 5 dosiert und getrocknet.
Die heißgeprägte Folie wird über Markierungen auf der Klebeschicht des Grundsensors fixiert. Das resultierende erforderliche Probevolumen beträgt ca. 0,5 µl.
Danach wird der Nutzen durch Schneiden in zehn Sensoren vereinzelt.

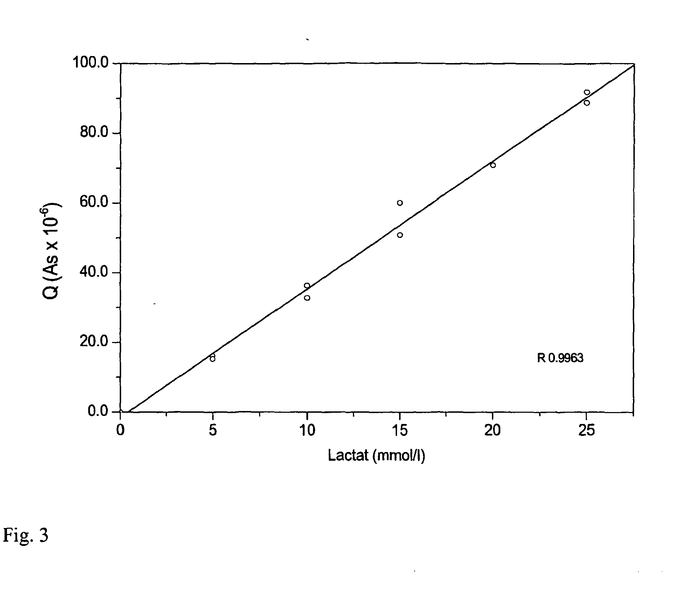

Zur Durchführung einer Lactatmessung wird der Sensor über die Kontaktbahnen 1c an eine potentiostatische Ausleseeinheit (Handmessgerät SensLab) mit einer Polarisationsspannung von +450 mV angeschlossen.
Durch Kontakt des Probeaufnahmekanals 3 mit einer Probelösung gelangen in weniger als einer Sekunde ca. 0,5 µl Probe bis in die Reaktions- und Messkammer 4. Aufgrund des hydrophobisierten Probestoppkanals 5 verbleibt die Probe in der Reaktions- und Messkammer 4, löst die Reaktionsschicht auf und generiert einen Messstrom, der über der Zeit von 10 sec integriert wird. Die Abhängigkeit des Messsignals von der Lactatkonzentration ist in Fig. 3 dargestellt.

### Beispiel 2:

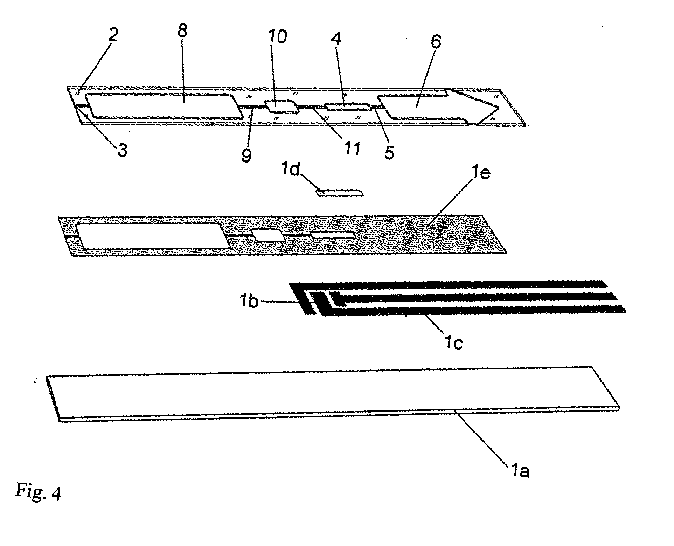

Erfindungsgemäßer Biosensor mit Reaktions- und Messkammeraufsatz 2 für den Einmalgebrauch zum Nachweis von p-Chinonderivatisierten Histamins. Zur Erläuterung dienen Fig. 4 bis Fig. 5.

Auf einen PVC Kunststoffsupport 1a mit einer Dicke von 0,6 mm werden in Zehnernutzen mittels Siebdruck nacheinander Elektrodenflächen 1b bzw. Kohlenstoffkontaktbahnen 1c (Acheson PE 401, Acheson NL), Isolationslack 1e (240 SB, ESL Europe) und Kleber (D 159, Kiwoprint) wie in Fig. 4 dargestellt aufgedruckt und jeweils bei 70°C gehärtet. Die Geometrie des Kleberfilms ist identisch mit der Geometrie der Isolationslackschicht 1e. Die Abmessungen eines Sensors sind 6 mm x 36 mm (B x L). Die Einzelflächen von Arbeits-, Referenz- und Gegenelektrode, die nacheinander angeordnet sind und die eigentliche Elektrodenfläche 1b ausmachen, betragen jeweils 1 mm². Mittels eines Dispensers werden 0,3 µl einer sekundären Reaktionslösung bestehend aus 20 µg Triton X 100 (Sigma) und 10 mg mikrokristalliner Zellulose (Aldrich) als Reaktionsschicht in 0,02 µl Dispensierschritten gleichmäßig über die gesamte Elektrodenfläche 1b verteilt aufgetragen.
Zur Herstellung des Reaktions- und Messkammeraufsatzes 2 dient eine mit einer photosensitiven Schicht modifizierte Polyamidfolie (PHAT-Film, Chromaline, Mnp.USA) von insgesamt 100 µm Dicke, deren Strukturierung über eine Filmvorlage durch Belichtung und der damit verbundenen Polymerisierung und anschließendem Auswaschen der unbelichteten Flächen erfolgt. Die als Zehnernutzen hergestellte Folie hat eine durchgängige Tiefe von 100 µm und weist wie in Fig. 4 dargestellt einen Probeeintrittskanal 3, eine Probeaufnahme- und Affinitätsreaktionskammer 8, einen ersten Übergangskanal 9, eine Enzymsubstratdepotkammer 10, einen zweiten Übergangskanal 11, eine Reaktions- und Messkammer 4, einen Probestoppkanal 5 und einen Probeauffangraum 6 auf.
Dementsprechend wurden Kanäle und Kompartimente bei identischen Tiefen mit folgenden Geometrien realisiert: Probeaufnahmekanal 3: 200 µm x 1 mm (B x L), Probeaufnahme- und Affinitätsreaktionskammer 8: 4 mm x 8 mm (B x L), erster Übergangskanal 9: 200 µm x 2 mm (B x L), Substrat- und Pufferdepotkammer 10: 1,5 mm x 1,5 mm (B x L), zweiter Übergangskanal 11: 200 µm x 2 mm (B x L), Reaktions- und Messkammer 4: 1 mm x 3 mm (B x L), Probestoppkanal 5: 200 µm x 1 mm und Probeauffangraum 6: 4 mm x 25 mm (B x L).

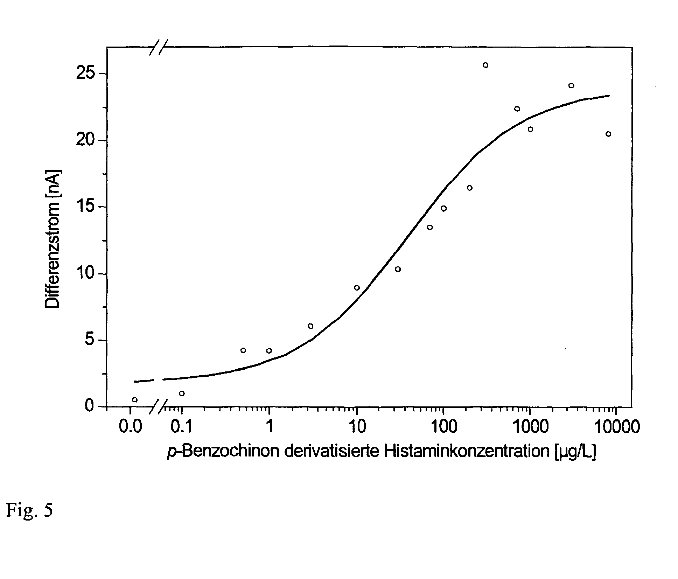

Mittels Dispenser werden nacheinander 10 nl einer 0,1%igen wässrigen Lösung aus mikrokristalliner Zellulose (Aldrich), die 0,1 % Triton X 100 (Sigma) und 2 % Polyethylenglycol (MG 8000) (Aldrich) enthält, in den Probeaufnahmekanal 3 und in den ersten Übergangskanal 9 aufgetragen. Nach dem Trocknen werden auf die Zelluloseschicht 15 nl einer 1%igen Chloroformlösung aus Paraffinwachs (Aldrich) dosiert und getrocknet.
In die Probeaufnahme- und Affinitätsreaktionskammer 8 und in die Reaktions- und Messkammer 4 werden jeweils nach Zwischentrocknung in 5 Dosierschritten nacheinander 20 µl Protein G (3 µg/ml, Sigma) und Antikörper gegen p-Chinonderivatisertes Histamin (5 µg/ml, BioTrend) auf die jeweilige Deckfläche aufgetragen und eine Antikörperimmobilisatschicht erzeugt. Danach werden 10 *µ*l β-Galactosidase-Histaminkonjugat (0,1 µg/ml) auf die Grundfläche der Probeaufnahme- und Affinitätsreaktionskammer 8 aufgetragen (β-Galactosidase, Calbiochem). In die Substrat- und Pufferdepotkammer 10 werden 10 µl einer p-Aminophenyl-ß-D-galactosidlösung (0,2 mM, Sigma) dosiert.
Die photolithographisch strukturierte Folie wird über Markierungen auf der Klebeschicht des Grundsensors fixiert. Das resultierende erforderliche Probevolumen beträgt ca. 4 µl.
Danach wird der Nutzen durch Schneiden in zehn Sensoren vereinzelt.
Zur Durchführung der Histaminbestimmung wird der Sensor über die Kontaktbahnen 1c an eine potentiostatische Ausleseeinheit (Handmessgerät SensLab) mit einer Polarisationsspannung von +200 mV vs. interner Referenzelektrode angeschlossen.
Durch Kontakt des Probeaufnahmekanals 3 mit einer Probelösung gelangen ca. 5 µl in die Probeaufnahme- und Affinitätsreaktionskammer 8.
Das in der Probe enthaltene p-Benzochinon-derivatisierte Histamin (beides Sigma) geht eine kompetitive Reaktion mit dem in der Probeaufnahme- und Affinitätsreaktionskammer 8 diffusionsfähig vorliegenden β-Galactosidase-Histaminkonjugat um die Bindungsstellen der in der Probeaufnahme- und Affinitätsreaktionskammer 8 immobilisierten Antikörperschicht ein. Nach definierter Inkubationszeit bzw. definierter Verzögerung des Probeflusses durch den ersten Verbindungskanal 9 gelangt die Probeflüssigkeit und damit auch das nicht gebundene β-Galactosidase-Histaminkonjugat zur Substrat- und Pufferdepotkammer 10, es wird das vorliegende p-Aminophenyl-β-D-galactosid gelöst und es beginnt die enzymatische Hydrolyse zu p-Aminophenol durch das Markerenzym. Die Probe gelangt nun über den zweiten Übergangskanal 11 in die sekundäre Reaktions- und Messkammer 4. Der nachfolgende Probestoppkanal 5 ist derart hydrophobisiert, dass die Kapillarkraftwirkung für einen Weitertransport der Probeflüssigkeit nicht ausreicht und die Probe innerhalb der Messzelle verbleibt. Dort werden die ß-Galactosidase-Histaminkonjugatmoleküle, die in der Probeaufnahme- und Affinitätsreaktionskammer 8 keine Bindung mit den immobilisierten Erkennungsmolekülen eingehen konnten, durch die Antikörperschicht, die direkt über den Elektrodenflächen 1b des Sensors fixiert ist, gebunden bzw. in der sekundären Reaktions- und Messkammer 4 angereichert. In Folge der weiteren Hydrolyse des p-Aminophenylgalactosids durch die β-Galactosidase wird p-Aminophenol angereichert und an der Arbeitselektrode bei +200 mV vs. interner Referenzelektrode oxidiert. Der Oxidationsstrom ist proportional der Histaminkonzentration in der Probe. Die Stromzunahme innerhalb der ersten Minute nach Probeflüssigkeitseintritt in die sekundäre Reaktions-und Messkammer 4 ist für unterschiedliche Histaminkonzentrationen in Fig. 5 dargestellt.

Es zeigen Fig. 1 bis Fig. 5:
- Fig. 1: Schnittdarstellung eines voltammetrischen Enzym-sensors mit kompartimentiertem Reaktions- und Messkammeraufsatz 2 entlang der Symmetrieachse zwischen Probeeintrittskanal 3 und Probeauffangraum 6, mit Kunststoffsupport 1a, mit Elektroden-flächen 1b, Enzym-Mediator-Reaktionsschicht 1d, Reaktions- und Messkammer 4, und Probestoppkanal 5.

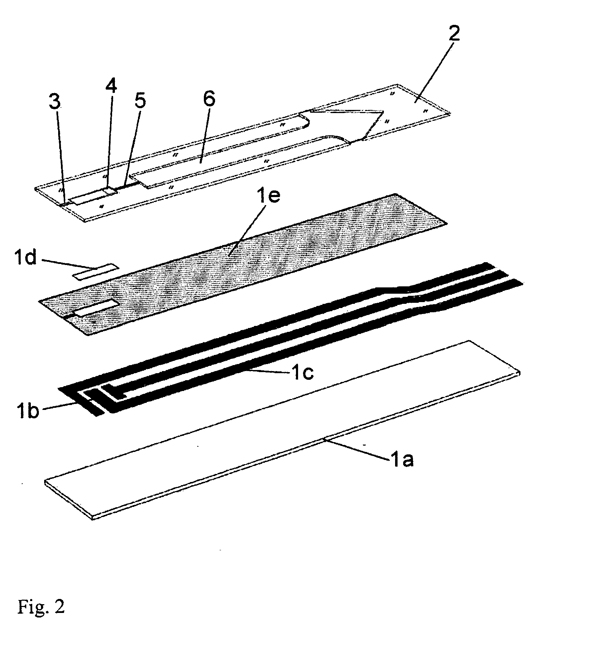

- Fig. 2: Dreidimensionale Darstellung eines voltammetrischen Enzymsensors mit kompartimentiertem Reaktions- und Messkammeraufsatz 2 und planarem Sensor 1, bestehend aus einem Support 1a, Elektrodenflächen 1b, Kontaktbahnen 1c einer Enzym-Mediator-Reaktionsschicht 1d und einer Dickschichtisolationsschicht 1e;
- Fig. 3: Abhängigkeit der coulometrischen Messwerte eines Lactatsensors von der Lactatkonzentration (Doppelbestimmungen) gemessen mit einem erfindungsgemäßen Enzymsensor der Fig. 1 und 2;
- Fig. 4: Dreidimensionale Darstellung eines voltammetrischen Immunosensors mit kompartimentiertem Reaktions- und Messkammeraufsatz 2, Kunststoffsupport 1a, Elektrodenflächen 1b mit Kontaktbahnen 1c, einer Dickschichtisolationsschicht 1e, sekundärer Reaktionsschicht 1d, Probeeintrittskanal 3, Probeaufnahme- und Affinitätsreaktionskammer 8 mit immobilisierter Antikörperschicht und markiertem Analyten, Verbindungskanal 20, Enzymsubstrat-depotkammer 10, Verbindungskanal 11, Reaktions-und Messkammer 4, Probestoppkanal 5 und Probeauffangraum 6 und
- Fig. 5: Abhängigkeit der amperometrischen Messwerte eines immunochemischen Histaminsensors von der Konzentration an p-Benzochinon-derivatisiertem Histamin gemessen mit einem erfindungsgemäßen Immunosensors der Fig. 4.

## Patentansprüche

1. Biosensor bestehend aus einem planaren Sensor oder Teststreifen (1) und einem kompartimentierten Reaktions- und Messkammeraufsatz (2), der einen hydrophilisierten Probeaufnahmekanal (3) mit Eintrittsöffnung, eine Reaktions- und Messkammer (4), deren mittlerer Querschnitt mindestens doppelt so tief und/oder breit ist wie der des Probeaufnahmekanals (3) und ein analyterkennendes Enzym- oder Enzymsystem sowie einen Elektronenmediator enthält, einen hydrophobisierten Probestoppkanal (5), der in der Tiefe und/oder Breite seines Querschnitts um die Hälfte bis Zwanzigfache geringer ist als die Tiefe und/oder Breite des Austrittsöffnungsguerschnitts der Reaktions- und Messkammer (4) ist, und einen großvolumigen Probeauffangraum (6) umfasst, dessen Querschnitt mindestens doppelt so tief und/oder breit wie der des Probestoppkanals (5) ist, wobei Probeaufnahmekanal (3), Reaktions- und Messkammer (4), Probestoppkanal (5) und Probeauffangraum (6) miteinander verbunden sind und der Reaktions- und Messkammeraufsatz (2) irreversibel mit dem Sensor oder Teststreifen (1) so verbunden ist, dass die Messkammer (4) über der sensitiven Fläche (1b;1d) des Sensors oder Teststreifens (1) positioniert ist.

2. Biosensor nach Anspruch 1,
**dadurch gekennzeichnet, dass**
der Probeaufnahmekanal (3) und der Probestoppkanal (5) ein Volumen von 0,005 bis 0,05 µl aufweisen, das Volumen der Reaktions- und Messkammer (4) von 0,1 bis 1,0 µl beträgt und das Volumen des Probeauffangraumes (6) mindestens 10 µl beträgt.

3. Biosensor nach Anspruch 1,
**dadurch gekennzeichnet, dass**
zwischen Probeaufnahmekanal (3) und Reaktions- und Messkammer (4) eine Probevorbehandlungskammer angeordnet ist.

4. Biosensor nach Anspruch 1,
**dadurch gekennzeichnet, dass**
der Probeaufnahmekanal (3) an den Innenflächen eine Tensidbeschichtung aufweist oder dass er ein hydrophilisiertes poröses Füllmaterial enthält.

5. Biosensor nach Anspruch 1,
**dadurch gekennzeichnet, dass**
der Probestoppkanal (5) an den Innenflächen eine hydrophobe Beschichtung aufweist oder dass er ein hydrophobisiertes poröses Füllmaterial enthält.

6. Biosensor nach Anspruch 1,
**dadurch gekennzeichnet, dass**
nach dem Probeaufnahmekanal (3) eine Probeaufnahme- und Affinitätskammer (8) angeordnet ist, die markierten Analyten enthält und an deren Innenflächen Erkennungsmoleküle immobilisiert sind, die Probeaufnahme- und Affinitätskammer (8) über einen hydrophobisierten Verbindungskanal (9) mit einer Substrat- und Pufferdepotkammer (10) verbunden ist und diese über einen Verbindungskanal (11) mit der Messkammer (4) verbunden ist, an deren Innenflächen Fängermoleküle immobilisiert sind.

7. Biosensor nach Anspruch 6,
**dadurch gekennzeichnet, dass**
das Volumen der Probeaufnahme- und Affinitätskammer (8) von 2 bis 20 µl beträgt, das Volumen der Substrat- und Pufferdepotkammer (10) von 0,1 1 µl bis 5 µl beträgt und die Messkammer (4) ein Volumen von 0,1 µl bis 5 µl aufweist.

8. Biosensor nach Anspruch 6,
**dadurch gekennzeichnet, dass**
der Verbindungskanal (9) an den Innenflächen eine hydrophobe Beschichtung aufweist oder dass er ein hydrophobisiertes poröses Füllmaterial enthält.

## Claims

1. A biosensor consisting of a planar sensor or test strip (1) and a compartmentalized reaction and measurement chamber attachment (2) which includes a hydrophilized sample-receiving channel (3) with inlet opening, a reaction and measuring chamber (4), the mean cross-section of which being at least twice as deep and/or wide as that of the sample-receiving channel (3), and an analyte-sensing enzyme or enzyme system as well as an electron mediator, and comprises a hydrophobized sample stop channel (5), the cross-sectional depth and/or width of which being smaller than the depth and/or width of the outlet opening cross-section of the reaction and measurement chamber (4) by a factor of two to twenty, and a large-volume sample-collecting space (6), the cross-section of which being at least twice as deep and/or wide as that of the sample stop channel (5), said sample-receiving channel (3), reaction and measurement chamber (4), sample stop channel (5) and sample-collecting space (6) being interconnected, and said reaction and measurement chamber attachment (2) being irreversibly attached to the sensor or test strip (1) in such a way that the measurement chamber (4) is positioned over the sensitive surface (1b; 1d) of the sensor or test strip (1).

2. The biosensor according to claim 1,
**characterized in that**
the sample-receiving channel (3) and the sample stop channel (5) have a volume of 0.005 to 0.05 µl, the volume of the reaction and measurement chamber (4) is 0.1 to 1.0 µl, and the volume of the sample-collecting space (6) is at least 10 µl.

3. The biosensor according to claim 1,
**characterized in that**
a sample pretreatment chamber is arranged between the sample-receiving channel (3) and the reaction and measurement chamber (4).

4. The biosensor according to claim 1,
**characterized in that**
the sample-receiving channel (3) has a surfactant coating on the inner surfaces thereof or includes a hydrophilized porous filling material.

5. The biosensor according to claim 1,
**characterized in that**
the sample stop channel (5) has a hydrophobic coating on the inner surfaces thereof or includes a hydrophobized porous filling material.

6. The biosensor according to claim 1,
**characterized in that**
a sample-receiving and affinity chamber (8) is arranged downstream of the sample-receiving channel (3), which chamber contains labelled analyte and has recognition molecules immobilized on the inner surfaces thereof, the sample-receiving and affinity chamber (8) is connected via a hydrophobized connecting channel (9) with a substrate and buffer storage chamber (10) which is connected via a connecting channel (11) with the measurement chamber (4) which has scavenger molecules immobilized on the inner surfaces thereof.

7. The biosensor according to claim 6,
**characterized in that**
the volume of the sample-receiving and affinity chamber (8) is 2 to 20 µl, the volume of the substrate and buffer storage chamber (10) is 0.1 to 5 µl, and the measurement chamber (4) has a volume of 0.1 to 5 µl.

8. The biosensor according to claim 6,
**characterized in that**
the connecting channel (9) has a hydrophobic coating on the inner surfaces thereof or includes a hydrophobized porous filling material.

## Revendications

1. Biocapteur constitué d'un capteur planaire ou d'une bandelette de test (1) et d'un montage compartimenté de chambre de réaction et de mesure (2) qui contient un canal de réception d'échantillon rendu hydrophile (3) avec une ouverture d'entrée, une chambre de réaction et de mesure (4), dont la section moyenne est au moins deux fois plus épaisse et/ou deux fois plus large que le canal de réception d'échantillon (3) et un enzyme ou un système enzymatique identifiant l'analyte ainsi qu'un médiateur d'électrons et qui comprend un canal d'arrêt d'échantillon (5) rendu hydrophobe, dont la section est de deux à vingt fois plus petite en épaisseur et/ou en largeur que la section de l'ouverture de sortie de la chambre de réaction et de mesure (4), et un compartiment de recueil d'échantillon (6) à grand volume, dont la section transversale est au moins deux fois plus épaisse et/ou plus large que le canal d'arrêt d'échantillon (5), moyennant quoi le canal de réception d'échantillon (3), la chambre de réaction et de mesure (4), le canal d'arrêt d'échantillon (5), et le compartiment de recueil d'échantillon (6) sont reliés l'un à l'autre et le montage de chambre de réaction et de mesure (2) est relié de manière irréversible au capteur ou à la bandelette de test (1) de telle sorte que la chambre de mesure (4) est positionnée au dessus de la surface sensible (1 b ; 1 d) du capteur ou de la bandelette de test.

2. Biocapteur selon la revendication 1,
**caractérisé en ce que**
le canal de réception d'échantillon (3) et le canal d'arrêt d'échantillon (5) ont un volume de 0,005 à 0,05 µl, le volume de la chambre de réaction et de mesure (4) est de 0,1 à 1,0 µl et le volume du compartiment de recueil d'échantillon (6) est au moins 10 µl.

3. Biocapteur selon la revendication 1,
**caractérisé en ce**
**qu'**une chambre de pré-traitement d'échantillon est disposée entre le canal de réception d'échantillon (3) et la chambre de réaction et de mesure (4).

4. Biocapteur selon la revendication 1,
**caractérisé en ce que**
le canal de réception d'échantillon (3) possède un revêtement tensioactif sur ses surfaces intérieures ou qu'il contient un matériau de charge poreux rendu hydrophile.

5. Biocapteur selon la revendication 1,
**caractérisé en ce que**
le canal d'arrêt d'échantillon (5) comprend un revêtement hydrophobe sur ses surfaces intérieures ou qu'il contient un matériau de charge poreux rendu hydrophobe.

6. Biocapteur selon la revendication 1,
**caractérisé en ce**
**qu'**après le canal de réception d'échantillon (3), est disposée une chambre de réception d'échantillon et d'affinité (8), qui contient des analytes marquées et sur les surfaces intérieures de laquelle des molécules de reconnaissance sont fixées, la chambre de réception d'échantillon et d'affinité (8) est reliée par l'intermédiaire d'un canal de jonction (9) rendu hydrophobe à une chambre de stockage de substrat ou de tampon (10) et celle-ci est reliée par l'intermédiaire d'un canal de jonction (11) à la chambre de mesure (4), sur les surfaces intérieures de laquelle des molécules capteurs sont fixées.

7. Biocapteur selon la revendication 6,
**caractérisé en ce que**
le volume de la chambre de réception d'échantillon et d'affinité (8) est de 2 à 20 µl, le volume de la chambre de dépôt de substrat et de tampon (10) est de 0,1 à 5 µl et la chambre de mesure (4) a un volume de 0,1 à 5 µl.

8. Biocapteur selon la revendication 6,
**caractérisé en ce que**
le canal de jonction (9) comporte sur les surfaces intérieures un revêtement hydrophobe ou qu'il contient un matériau de charge poreux rendu hydrophobe.
